# EUROPEAN PATENT SPECIFICATION

(11) **EP 1 224 911 B1**
(45) Date of publication and mention of the grant of the patent: **02.11.2006**
(21) Application number: 01200184.8
(22) Date of filing: 19.01.2001
(51) Int. Cl.: A61B 17/08, A61B 17/68

(54) **Device for fastening a cranial flap to the cranial vault**
Vorrichtung zur Befestigung eines einer Schädelkapsel entnommenen Kallottensegments
Dispositif de fixation pour segment cranien

(43) Date of publication of application: 24.07.2002
(73) Proprietor: Acampora, Sergio, 80122 Napoli (IT); Gazzani, Romolo Igino, 15069 Serravalle Scrivia (Alessandria) (IT)
(72) Inventor: Acampora, Sergio, 80122 Napoli (IT); Gazzani, Romolo Igino, 15069 Serravalle Scrivia (Alessandria) (IT)
(74) Representative: Martegani, Franco

(56) References cited:
- EP-A- 0 873 718
- WO-A-98/29040
- DE-C- 19 634 697
- US-A- 5 474 557
- US-A- 5 800 436

## Description

This invention refers to a device for fastening a cranial flap to the cranial vault.

As known, a craniotomy, meaning the incision and cutting out of a bone flap from the cranial vault, is a mandatory neurosurgical procedure for the treatment of any inter-cranial damage.

This procedure is generally carried out in the following manner: after properly positioning the patient depending on the intended action and planning the flap, the scalp is disinfected, the skin incising outline is drawn up and the head is covered with sterile linen.

The skin incision is performed in short sections, while controlling the hemostasis by bipolar coagulation and the application of skin clips.

After the incision has been completed, the flap of scalp is detached from the underlying pericranium, and the pericranium and muscle are incised by a diathermic cut along the intended bone flap outline, except for occasionally leaving a shaft to allow for a certain blood flow to the bone.

In certain flaps, especially those of a frontal or pterional type, the dissociation of these planes may be avoided, so as to spare the nerve endings of the facial nerve.

The bone flap is thereupon incised by drilling one or several holes, depending on whether a cutting blade or a drill bit on a pneumatic drill is applied, so that the underlying cranial vault may be detached free hand by the key hole while simultaneously incising the bone, or the hard part of the bone is dissected between one drill hole and the next by using a curved periosteum detacher. In the following, a sawing wire is guided through and pulled upward by hand at the extremities, thus excising the bone in individual segments.

The bone flap is wrapped in moist gauze and kept apart from the operating area by some fastening devices if provided with a shaft, or left free in a physiological solution in a cup.

At the end of the neurosurgical operation, after the cranial vault has been sutured and the bone edges have been turned up, the flap is refitted into the aperture and fastened with separate metal points or wires passed through small drill holes provided in pairs along the free edge of the cranial bone.

The cranial flap is occasionally left entirely free, except for a thread of silk applied on the cranial vault, passed through two drill holes and tied to the center of the bone flap.

It is nevertheless evident that solutions of this type cannot generally afford an esthetically acceptable seal, as they are not always capable of preventing the bone flap from emerging, curving, sloping or turning.

In order to prevent these drawbacks, some micro-platelets made of titanium have more recently been applied by threading them in at three points on the bone flap and along the edge of the cranial vault.

Nevertheless, even this solution suffers from the fact that these micro-platelets made of titanium are not sufficiently elastic to ensure that an excess pressure will not arise on the brain during the post-operation period.

An other example of a device for fastening a cranial flap to the cranial vault is disclosed in US-A-5800436, wherein two disks, each provided with a row of teeth, are mounted on a pin facing each other to fix the bone.

The purpose of this invention is therefore to produce a device for fastening a cranial flap to the cranial vault, capable of ensuring a correct ossification of the bone flap along the edge of the cranial vault.

Another purpose of the invention is to produce a device for fastening a cranial flap to the cranial vault, capable of ensuring a lower pressure on the brain itself, even if a cerebral edema should develop during the post-operation period.

These and other purposes are achieved by a device for fastening a cranial flap to the cranial vault, according to claim 1, which is being referred to for brevity.

Other characteristics of the invention are defined in the additional claims attached to this patent application.

Further scopes and advantages of this invention will become clear from the description and attached drawings to follow, supplied for purely explanatory and nonlimiting purposes, in which:
- Figure 1 shows a view of the device for fastening a cranial flap to the cranial vault, according to this invention, in a closed configuration,
- Figure 2 shows a view of the device of Figure 1, in an open configuration,
- Figure 3 shows a view of the device of the invention in a first phase of its application,
- Figure 4 shows a view of the device of the invention in a second phase of its application, and
- Figure 5 shows a view of the device of the invention in a further phase of its application.

With particular reference to the figures mentioned above, the device for fastening a cranial flap to the cranial vault according to this invention is indicated in its overall form by reference to the number 10. The device 10 includes an upper ring 11 and a lower ring 12, connected to each other by a pillar 13.

The entire structure of the device 10 is made from a single wire; this particular design provides for an opened upper ring 11, composed in particular of two sections 18 and 19, and for a closed lower ring 12.

Moreover, the fact that the structure of the device 10 is made from a single wire achieves a pillar 13 be constituted by two adjacent sections of wire.

The elasticity of the pillar 13 connecting the two rings 11 and 12 makes it possible to utilize only a few sizes in relation to the bone thickness 20, for example only three sizes, while its circular form allows any rotating motion, including a shifting of the rings out of a parallel plane.

The wire used to produce the device 10 is preferably made of a nickel-titanium alloy with a memorized form.

Going into further detail in relation to the applications of the cranial fastening device 10 of this invention, it is worth noting that the instrument specialist sets up a number of devices 10 to handle a craniotomy in various sizes, by placing them into a small cup (not shown) containing a chilled physiological solution, for instance at a temperature of -8°C.

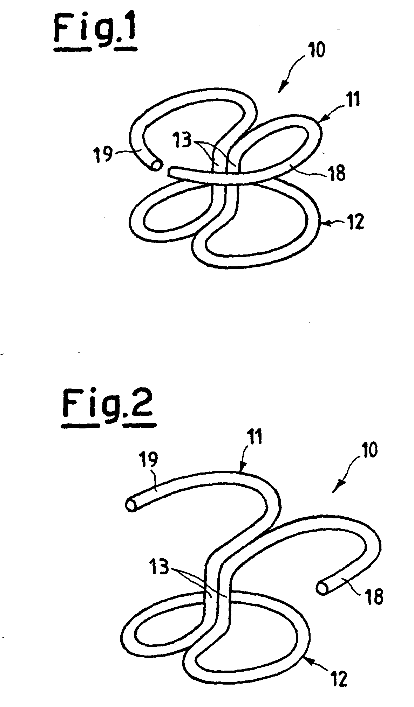

The instrument specialist grasps the upper ring 11 with a grooved clamping forceps and then laterally twists the sections 18 and 19 of the ring 11 by a knurled forceps, until opening them as shown in Figure 2.

Using the clamping forceps, the surgeon then inserts the device 10 so that the pillar 13 comes in contact with the bone edge 20, while passing the lower ring 11 through the space between the bone 15 and the cranial vault 16.

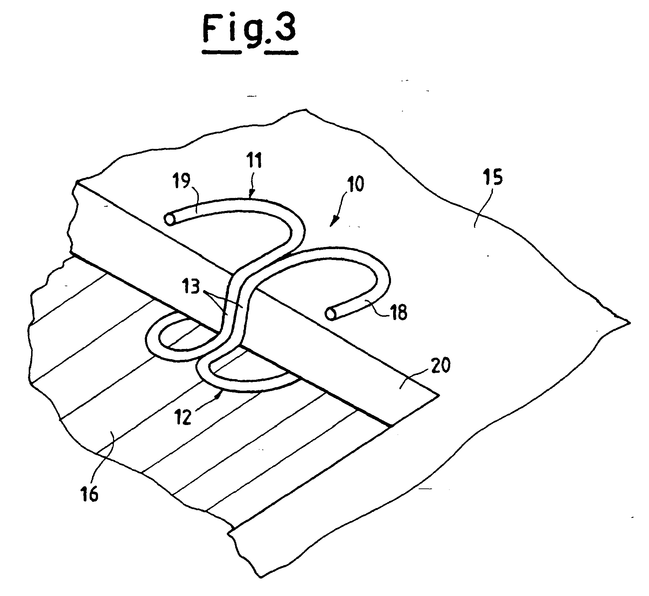

These operations are carried out by choosing the sizes most suited to the thickness of the bone 15 and the most appropriate number of devices 10, in relation to the size and shape of the craniotomy, as shown in Figure 3.

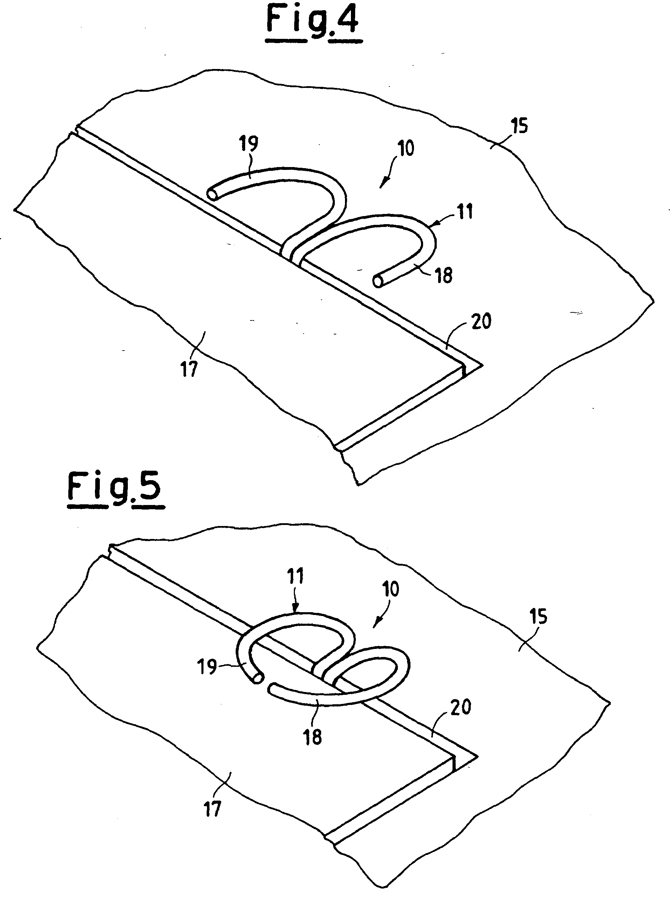

The bone operculum 17 is rested on the lower ring 12 of the device 10, as shown in Figure 4.

The surgeon then verifies the proper position of the operculum 17, irrigates the devices 10 with a hot physiological solution (for example at a temperature of 45°C), until the upper ring 11 is fully closed; this operation is shown in Figure 5.

The cranial fastening device 10 of this invention is suitable for any craniotomy, as its shape and the various sizes in which it can be produced based on the bone thickness allow it to adhere to the cranial flap at the edge of the cranial vault, both at the inner and outer perimeter, so as to permit a proper ossification process.

The device 10 can be utilized to fasten a volé inserted into the cranial vault instead of for instance resorting to the use of micro-plates and screws.

At the same time, the extreme elasticity of the nickel-titanium alloy guarantees a lower pressure on the brain itself during the post-operation period, when edemas and therefore brain swelling phenomena may occur.

The above description clearly outlines the characteristics as well as the advantages of the device for fastening a cranial flap to the cranial vault as an object of this invention.

It is also evident that numerous variants may be applied to the device for fastening a cranial flap to the cranial vault, as an object of this invention, without thereby abandoning the principles of novelty inherent in the inventive idea.

Finally and in the practical implementation of the invention, the materials, shapes and sizes of the details outlined above may be of any kind depending on the requirements, and the same may be substituted by others of a technically equivalent type.

## Claims

1. A device (10) for fastening a cranial flap (17) to the cranial vault (16), **characterized in that** it is made from a simple wire that is shaped such that an upper ring like section (11) is provided, having an opening where the ends of the wire meet thus forming two sections (18, 19), and a closed lower ring like section (12) connected to the upper ring like section (11) by a pillar (13) constituted by two adjacent sections of wire, where said upper ring like section (11) is composed of two bendable sections (18, 19) and may assume a first configuration in which the mentioned sections (18, 19) are approached and the ends of the wire are in close proximity, and a second configuration in which the mentioned sections (18, 19) are spread apart.

2. A device (10) according to claim 1,
**characterized in that** said pillar (13) may be placed in contact with the bone edge (20), by passing the lower ring (11) through the space between the bone (15) and the cranial vault (16), so that a bone flap (17) may subsequently be rested on said lower ring (12).

3. A device (10) according to claim 2,
**characterized in that** said sections (18, 19) of the upper ring (11) may be rotated laterally until opened, so as to allow the mentioned bone flap (17) to be rested on the same, and subsequently approached until the upper ring (11) is closed, so as to hold the mentioned bone flap (17), while irrigating them with a hot physiological solution.

4. A device (10) according to claim 1,
**characterized in that** said single wire is in a material having a memorized shape.

5. A device (10) according to claim 4,
**characterized in that** said wire is made of a nickel-titanium alloy.

## Patentansprüche

1. Vorrichtung (10) zur Befestigung einer Schädelklappe (17) an der Schädelhöhle (16),
**dadurch gekennzeichnet,**
**dass** sie aus einem einzigen Draht gefertigt ist, welcher derart geformt ist, dass ein oberer ringartiger Abschnitt (11) bereitgestellt ist, welcher eine Öffnung aufweist, wo die Enden des Drahts sich treffen, wodurch zwei Abschnitte (18, 19) ausgebildet werden, und ein geschlossener unterer ringartiger Abschnitt (12), welcher mit dem oberen ringartigen Abschnitt (11) durch eine Säule (13) verbunden ist, welche durch zwei benachbarte Drahtabschnitte gebildet ist, wobei der obere ringartige Abschnitt (11) aus zwei biegbaren Abschnitten (18, 19) zusammengesetzt ist und eine erste Konfiguration, in welcher die genannten Abschnitte (18, 19) angenähert sind und die Enden des Drahts eng benachbart sind, und eine zweite Konfiguration, in welcher die genannten Abschnitte (18, 19) auseinandergespreizt sind, einnehmen kann.

2. Vorrichtung (10) nach Anspruch 1,
**dadurch gekennzeichnet,**
**dass** die Säule (13) in Kontakt mit der Knochenkante (20) positioniert werden kann, indem der untere Ring (11) durch den Raum zwischen dem Knochen (12) und der Schädelhöhle (16) geführt wird, so dass eine Knochenklappe (17) anschließend auf den unteren Ring (12) gelegt werden kann.

3. Vorrichtung (10) nach Anspruch 2,
**dadurch gekennzeichnet,**
**dass** die Abschnitte (18, 19) des oberen Rings (11) bis zur Öffnung seitlich gedreht werden können, um es der genannten Knochenklappe (17) zu ermöglichen, auf denselben gelegt zu werden, und anschließend angenähert werden können, bis der obere Ring (11) geschlossen ist, um die genannte Knochenklappe (17) zu halten, während sie mit einer warmen physiologischen Lösung gespült werden.

4. Vorrichtung (10) nach Anspruch 1,
**dadurch gekennzeichnet,**
**dass** der Einzeldraht aus einem Material ist, welches eine gespeicherte Form aufweist.

5. Vorrichtung (10) nach Anspruch 4,
**dadurch gekennzeichnet,**
**dass** der Draht aus einer Nickel-Titan-Legierung hergestellt ist.

## Revendications

1. Dispositif (10) de fixation d'un segment crânien (17) à la voûte crânienne (16), **caractérisé en ce qu'**il est constitué d'un simple câble qui est formé de sorte qu'une section supérieure semblable à un anneau (11) soit ménagée, ayant une ouverture où les extrémités du câble se rencontrent en formant ainsi deux sections (18, 19), et une section inférieure semblable à un anneau (12), reliée à la section supérieure semblable à un anneau (11) par le biais d'une colonne (13) constituée de deux sections adjacentes de câbles, où ladite section supérieure semblable à un anneau supérieure (11) est composée de deux sections pliables (18, 19) et peut prendre une première configuration, dans laquelle les sections mentionnées (18, 19) sont rapprochées et les extrémités du câble sont très proches et une seconde configuration dans laquelle les sections mentionnées (18, 19) sont éloignées.

2. Dispositif (10) selon la revendication 1, **caractérisé en ce que** ladite colonne (13) peut être placée en contact avec le bord de l'os (20), en passant l'anneau inférieur (11) à travers l'espace entre l'os (15) et la voûte crânienne (16), de sorte qu'un segment osseux (17) puisse ensuite être appuyé sur ledit anneau inférieur (12).

3. Dispositif (10) selon la revendication 2, **caractérisé en ce que** lesdites sections (18, 19) de l'anneau supérieur (11) peuvent être tournées latéralement jusqu'à ouverture, de façon à permettre au segment osseux mentionné (17) de s'y appuyer, puis approchées jusqu'à ce que l'anneau supérieur (11) soit fermé, de façon à maintenir le segment osseux mentionné (17), tout en les irriguant avec une solution physiologique chaude.

4. Dispositif (10) selon la revendication 1, **caractérisé en ce que** ledit câble simple est réalisé en un matériau à mémoire de forme.

5. Dispositif (10) selon la revendication 4, **caractérisé en ce que** ledit câble est constitué d'un alliage de nickel-titane.
